# EUROPEAN PATENT APPLICATION

(11) **EP 4 174 556 A1**
(43) Date of publication of application: **03.05.2023**
(21) Application number: 21875059.4
(22) Date of filing: 03.09.2021
(51) Int. Cl.: G02B 23/24, A61B 1/012, A61B 1/018

(54) **ENDOSCOPE**

(30) Priority: 29.09.2020 JP 2020163438
(71) Applicant: HOYA CORPORATION, Shinjuku-ku Tokyo 160-8347 (JP)
(72) Inventor: MORISHIMA, Takayoshi, Tokyo 160-8347 (JP); IKETANI, Kohei, Tokyo 160-8347 (JP); ITO, Keiji, Tokyo 160-8347 (JP)
(74) Representative: Lucke, Andreas
(86) International application number: PCT/JP2021/032379
(87) International publication number: WO 2022/070757

(57) **Abstract**

An endoscope capable of both improving accuracy of position control of a treatment tool and ensuring a gap for sucking a liquid is provided.

The endoscope includes an insertion portion having a round tubular shape to be inserted into a body lumen, and includes: a treatment tool passage (78) having one end opened on a distal tip surface (131) of the insertion portion, penetrating the insertion portion in an axial length direction, and allowing a treatment tool to pass through; and a fluid passage (79) having one end opened on the distal tip surface of the insertion portion, penetrating the insertion portion in the axial length direction, and allowing a fluid that has flowed from the one end to flow through, in which the treatment tool passage (78) and the fluid passage (79) are partially continuous in a cross-sectional view.

## Description

### Technical Field

The present invention relates to an endoscope including an insertion portion in a round tubular shape to be inserted into a body lumen.

The present application claims priority based on Japanese Patent Application No. 2020-163438 filed on September 29, 2020, the entire contents of which are incorporated herein by reference.

### Background Art

Conventionally, an endoscope, in which an insertion portion to be inserted into a body lumen includes a first bending section and a second bending section sequentially from a distal tip side, is widely used.

For example, Patent Literature 1 discloses an endoscope in which an imaging device having an imaging unit including expensive component parts, such as an image sensor, an imaging board, and an imaging lens, is made removable and collectable from an insertion portion of the endoscope, so that the collected imaging unit is made reusable.

In addition, Patent Literature 2 discloses an endoscope in which a gap between an inner circumferential surface of a channel and a treatment tool is configured to be changeable in accordance with an outer diameter of the treatment tool to be inserted into the channel, so that the gap between the inner circumferential surface of the channel and the treatment tool is reduced.

For example, Patent Literature 3 discloses an endoscope in which a channel is formed in an oval shape or a semicircular shape, so that a fluid can be sucked from a gap between a treatment tool and an inner wall of the channel, when the treatment tool is inserted into the channel.

In addition, Patent Literature 4 discloses an endoscope in which a bending section that bends at the same dimension as a curvature radius of a channel tube is provided in an insertion portion regardless of a bending direction, so that the operability of the insertion portion is improved.

### Citation List

### Patent Literature

Patent Literature 1: JP 2018-228334 A
Patent Literature 2: WO 2020/017245 A
Patent Literature 3: JP H05-57847 A
Patent Literature 4: JP 6223218 B2

### Summary of Invention

### Technical Problem

In an endoscope having a configuration in which one channel is included, and a liquid such as a body fluid is sucked from a gap between the treatment tool and an inner wall of the channel when the treatment tool is inserted into the channel, it is necessary to ensure the gap for sucking the liquid.

However, there is a drawback that in a case where the inner diameter of the channel is increased by giving priority to the gap for sucking the liquid, the accuracy of the position control of the treatment tool inside the channel is insufficient, and in a case where the inner diameter of the channel is reduced by giving priority to the accuracy of the position control of the treatment tool, the gap for sucking the liquid is narrowed. In addition, a channel for the treatment tool and a channel for suction can be individually provided, but in this case, there is a drawback that the configuration of the insertion portion becomes complicated.

In the endoscopes of Patent Literature 1 to Patent Literature 3, no measures are taken for such drawbacks, and the drawbacks cannot be addressed.

In an endoscope including a through passage that penetrates the insertion portion in an axial length direction to suck a liquid, it is necessary to increase an inner diameter of such a through passage in order to increase a suction amount of the liquid. However, in a case where the inner diameter of the through passage is enlarged, the diameter of the insertion portion increases, and a burden is imposed on a subject at the time of a medical examination with use of the endoscope.

Nevertheless, in the endoscopes of Patent Literature 1 and Patent Literature 4, no measures are taken for such a drawback, and the drawback cannot be addressed.

The present invention has been made in view of such circumstances, and has an object to provide an endoscope capable of both improving the accuracy of position control of a treatment tool and ensuring a gap for sucking a liquid.

The present invention has been made in view of such circumstances, and has an object to provide an endoscope capable of suppressing an increase in diameter of an insertion portion, while enlarging an inner diameter of a through passage such as a channel.

### Solution to Problem

An endoscope according to the present invention includes an insertion portion having a round tubular shape to be inserted into a body lumen, and includes: a treatment tool passage having one end opened on a distal tip surface of the insertion portion, penetrating the insertion portion in an axial length direction, and allowing a treatment tool to pass through; and a fluid passage having one end opened on the distal tip surface of the insertion portion, penetrating the insertion portion in the axial length direction, and allowing a fluid that has flowed from the one end to flow through, in which the treatment tool passage and the fluid passage are partially continuous in a cross-sectional view.

In the present invention, the treatment tool passage and the fluid passage are partially continuous in the cross-sectional view. Therefore, stable liquid suction through the treatment tool passage and an improvement in accuracy of position control of the treatment tool are both achievable, regardless of the presence or absence of insertion of a treatment tool, with a compact configuration as compared with a case where the treatment tool passage and the fluid passage are separately provided.

An endoscope according to the present invention includes an insertion portion having a tubular shape to be inserted into a body lumen, in which a main portion having a circular shape in a cross-sectional view; an extension portion extending in a radial direction at one position of an outer circumference of the main portion; and a through passage at the one position, the through passage penetrating the insertion portion in an axial length direction, are formed.

In the present invention, the through passage can be configured to have a wide inner diameter including a part (the one position) corresponding to the outer circumference of the main portion, and the vicinity of the through passage is reinforced by the extension portion. Such partial reinforcement suppresses the diameter of the endoscope from being enlarged as a whole.

### Advantageous Effects of Invention

According to the present invention, an improvement in the accuracy of the position control of the treatment tool and ensuring of the gap for sucking the liquid are both achievable.

According to the present invention, an increase in diameter of an insertion portion can be suppressed, while an inner diameter of a through passage such as a channel is being enlarged.

### Brief Description of Drawings

Fig. 1 is an external view of an endoscope according to a first embodiment of the present invention.
Fig. 2 is a diagram illustrating a distal tip surface of a distal tip.
Fig. 3 is a cross-sectional view taken along line III-III in Fig. 2.
Fig. 4 is a cross-sectional view taken along line IV-IV in Fig. 2.
Fig. 5 is a cross-sectional view schematically illustrating a configuration of an imaging device.
Fig. 6 is an explanatory diagram for describing a connection between a cable unit and a relay portion.
Fig. 7 is a diagram illustrating a first relay board.
Fig. 8 is a diagram for describing the size of the first relay board.
Fig. 9 is a cross-sectional view taken along line IX-IX in Fig. 4.
Fig. 10 is an explanatory diagram for describing a method for collecting the imaging device from the endoscope.
Fig. 11 is an explanatory diagram for describing the method for collecting the imaging device from the endoscope.
Fig. 12 is an explanatory diagram for describing the method for collecting the imaging device from the endoscope.
Fig. 13 is an explanatory diagram for describing the method for collecting the imaging device from the endoscope.
Fig. 14 is a diagram illustrating a distal tip surface of a distal tip in an endoscope according to a second embodiment.
Fig. 15 is a cross-sectional view taken along line XV-XV in Fig. 14.
Fig. 16 is a cross-sectional view taken along line XVI-XVI in Fig. 15.
Fig. 17 is a diagram illustrating a distal tip surface of a distal tip in an endoscope according to a third embodiment.
Fig. 18 is a cross-sectional view taken along line XVIII-XVIII in Fig. 17.
Fig. 19 is a diagram illustrating a distal tip surface of a distal tip in an endoscope according to a fourth embodiment.
Fig. 20 is a cross-sectional view taken along line XX-XX in Fig. 19.

### Description of Embodiments

Hereinafter, an endoscope according to embodiments of the present invention will be described in detail with reference to the drawings.

### (First Embodiment)

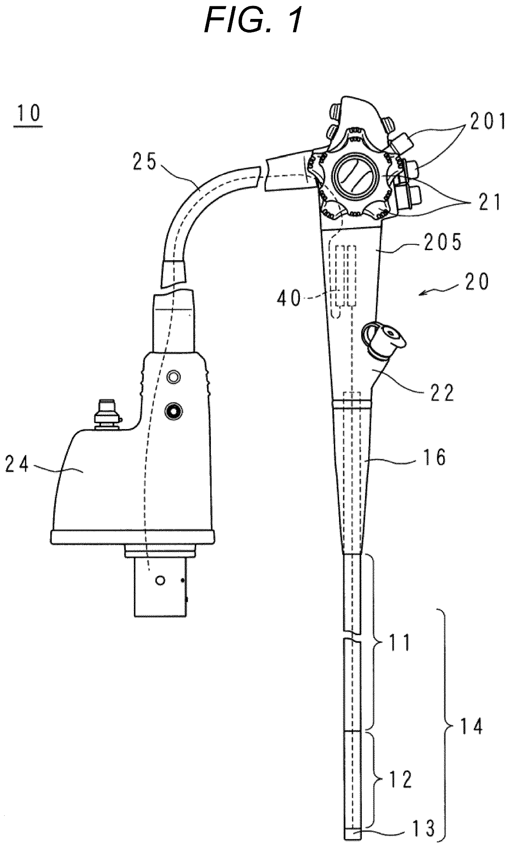

Fig. 1 is an external view of an endoscope 10 according to a first embodiment of the present invention. The endoscope 10 in the present embodiment is a so-called disposable endoscope.

The endoscope 10 includes an insertion portion 14 to be inserted into a body lumen of a subject, an operation unit 20 for operating the insertion portion 14, and a connector unit 24 connected with a processor, an air supply/water supply device, and the like, which are not illustrated.

The insertion portion 14 is connected with the operation unit 20 through a bend preventing portion 16, and the connector unit 24 is connected with the operation unit 20 through a universal cord 25.

The universal cord 25 has pliability, and includes an electric line that sends an electric signal from an imaging means of the insertion portion 14 to the connector unit 24, and a water passage through which water supplied from the connector unit 24 passes and an air passage through which air passes.

The operation unit 20 includes a grip portion 205, a button 201 for receiving an instruction from a user, and a bending knob 21 for operating bending of an active bending section 12 to be described later.

The grip portion 205 has a substantially tubular shape, and a channel inlet 22, into which a treatment tool or the like is to be inserted, is provided near the bend preventing portion 16 side. In addition, a relay portion 40, which connects an imaging unit 617 of a distal tip 13 to be described later with the processor, is incorporated inside the grip portion 205.

The insertion portion 14 has a tubular shape with a small diameter, and is configured to be bendable. The insertion portion 14 includes the distal tip 13, an active bending section 12, and a passive bending section 11 sequentially in this order from the distal tip side.

The distal tip 13 includes an imaging unit 617, to be described later, including an imaging means such as a charge coupled device (CCD) or a complementary metal oxide semiconductor (CMOS), a circuit board for driving the imaging means, an observation optical system, and the like, and a lens unit 62, to be described later, including a series of lens sets. In addition, the distal tip 13 includes an irradiation and/or illumination optical system or the like for irradiating an observation target site in the body lumen. An electric signal from the imaging unit 617 of the distal tip 13 is sent to the processor through the relay portion 40 and the connector unit 24.

The active bending section 12 is actively bendable. That is to say, the active bending section 12 is bent in four directions in accordance with an operation of the bending knob 21. On the other hand, the passive bending section 11 is passively bent. That is to say, the passive bending section 11 is bent in any of the four directions by contact with a target object.

The active bending section 12 and the passive bending section 11 are made of, for example, a multi-lumen tube, and are integrally formed.

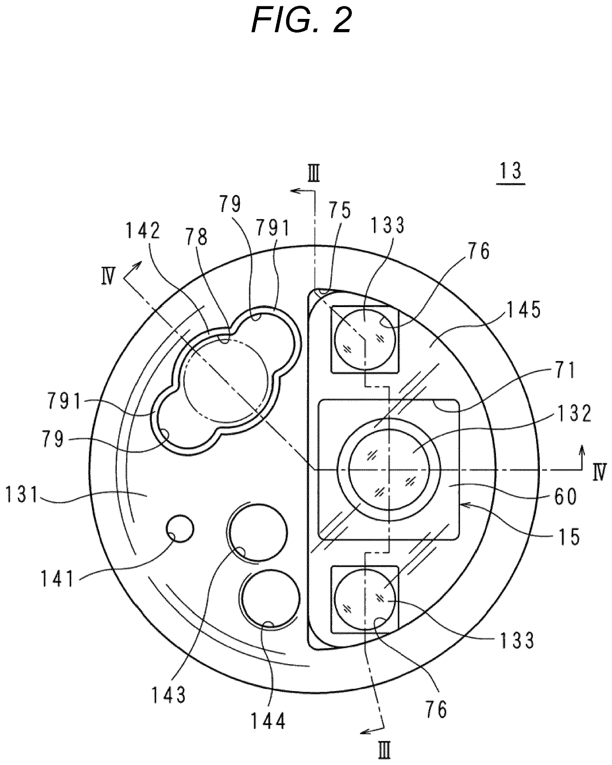

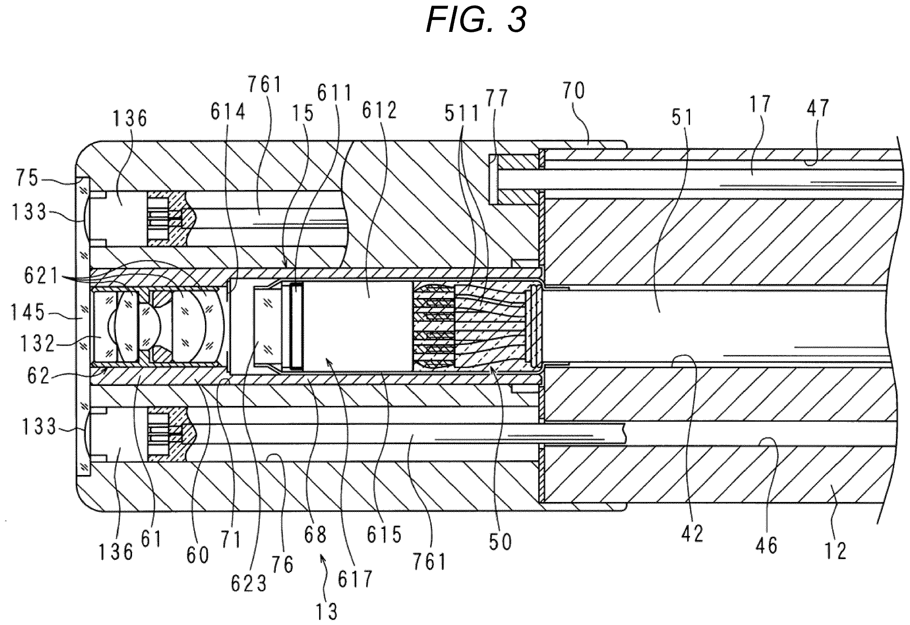

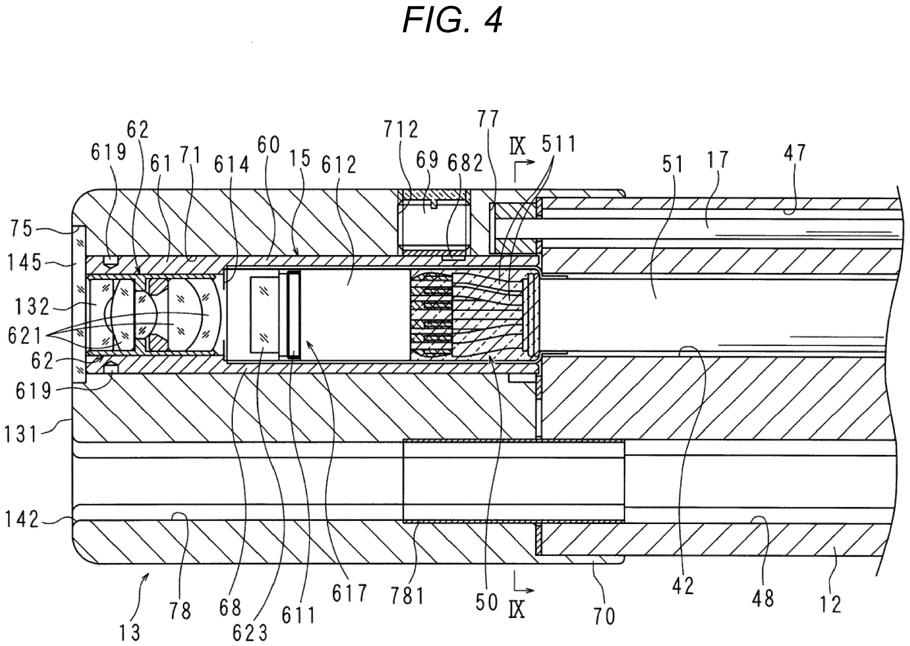

Fig. 2 is a diagram illustrating a distal tip surface 131 of the distal tip 13, Fig. 3 is a cross-sectional view taken along line III-III in Fig. 2, and Fig. 4 is a cross-sectional view taken along line IV-IV in Fig. 2.

The distal tip 13 has a columnar shape, and has a diameter larger than that of the active bending section 12. In the distal tip 13, a flange portion 70 is provided along the circumferential edge on an end surface on the active bending section 12 side. The distal tip of the active bending section 12 is internally fit with the flange portion 70.

In the endoscope 10, an imaging device 15 is incorporated in the insertion portion 14 and the bend preventing portion 16. The imaging device 15 includes the lens unit 62 and the imaging unit 617, and the imaging unit 617 is connected with the relay portion 40 through a cable unit 50.

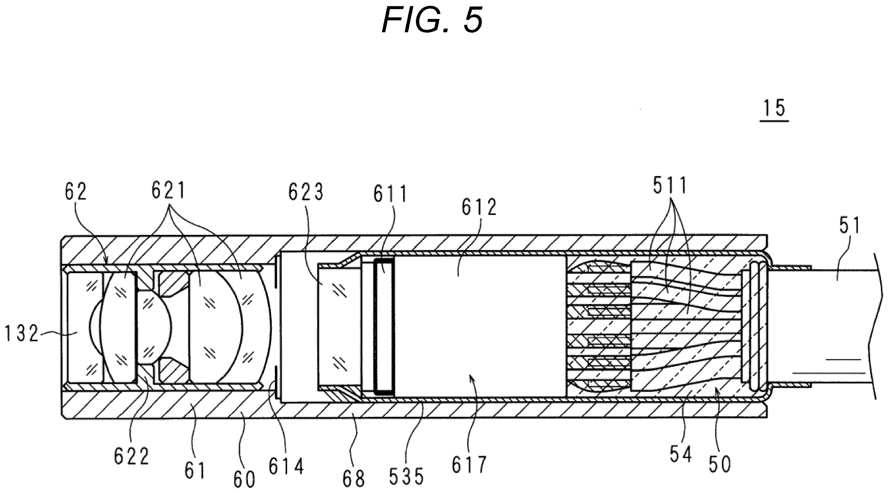

Fig. 5 is a cross-sectional view schematically illustrating a configuration of the imaging device 15.

The imaging device 15 includes a housing tube 60, and houses the lens unit 62 and the imaging unit 617. The housing tube 60 is a quadrangular tube, one end portion is a circular hole portion 61 having a circular inner circumferential surface, and the other part is a quadrangular hole portion 68 having a quadrangular inner circumferential surface. The lens unit 62 is inserted into the circular hole portion 61, and the imaging unit 617 is inserted into the quadrangular hole portion 68. A light shielding mask 614 is provided between the lens unit 62 and the imaging unit 617.

The lens unit 62 includes an observation window 132 and a plurality of imaging lenses 621. The observation window 132 and the plurality of imaging lenses 621 are coaxially disposed. Outer circumferential surfaces of the observation window 132 and the plurality of imaging lenses 621, in a state in which focus adjustment is made, are covered with and secured by a lens frame 622.

The imaging unit 617 includes a filter 623, an image sensor 611, and an imaging board 612.

The filter 623 removes unnecessary light such as infrared rays from the light that enters the image sensor 611. The light that enters from the lens unit 62 forms an image on the image sensor 611, and the image sensor 611 converts an optical image into an electric signal. In the imaging board 612, a driver circuit that controls the image sensor 611 is embedded. The cable unit 50 is connected with the imaging board 612 from the other end side of the housing tube 60.

The cable unit 50 includes a plurality of cable strands 511 bundled by a cable tube 51. One end of the cable unit 50 is connected with the imaging board 612. A connection between one end of the cable unit 50 and the imaging board 612 is potted with an insulating resin 54.

One end portions of the filter 623, the image sensor 611, the imaging board 612, and the cable unit 50 are held, in an assembled state, by an insulating tube 535. The insulating tube 535 is a heat shrinkable tube, and is shrunk by heat to secure each component part, so that the assembled state is maintained.

The cable unit 50 extends outward from the other end of the housing tube 60. The other end of the cable unit 50 is connected with the relay portion 40 (Fig. 1).

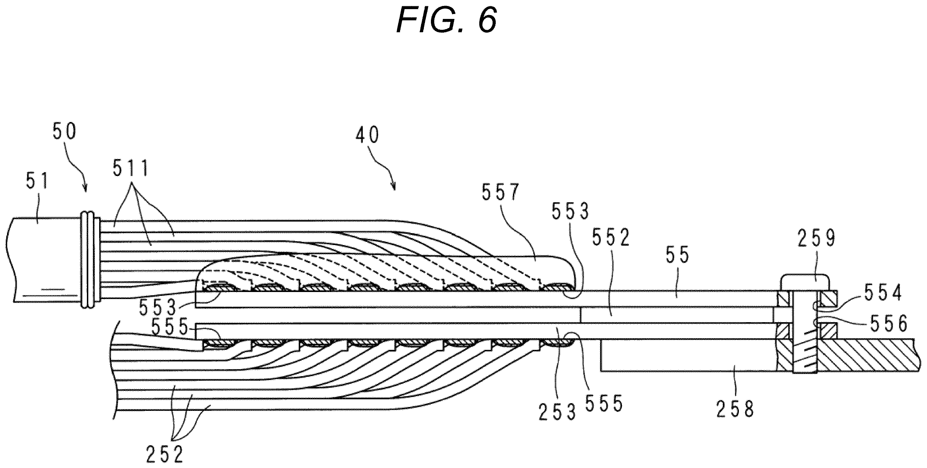

Fig. 6 is an explanatory diagram for describing a connection between the cable unit 50 and the relay portion 40. The relay portion 40 includes a first relay board 55 with which the other end of the cable unit 50 is connected, and a second relay board 253 with which the plurality of cable strands 252 are connected. The first relay board 55 includes a connection connector 552, and each cable strand 252 is connected with the processor through the universal cord 25 and the connector unit 24. The first relay board 55 and the second relay board 253 are disposed to face each other, a connection connector 552 is interposed therebetween, and the first relay board 55 and the second relay board 253 are connected with each other through the connection connector 552.

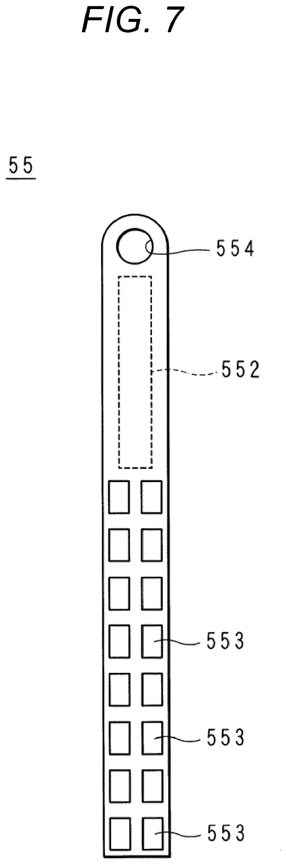

Fig. 7 is a diagram illustrating the first relay board 55. In Fig. 7, the connection connector 552 is indicated by a dotted line for convenience of description.

The first relay board 55 has a substantially rectangular shape, and a corner of one end portion is R-chamfered. A circular through hole 554 is formed at such one end portion of the first relay board 55.

In addition, in the first relay board 55, a rectangular land portion 553 is formed from the other end to an intermediate portion in the longitudinal direction. A pair of land portions 553 are formed in the width direction of the first relay board 55, and a plurality of the pair of land portions 553 are disposed in parallel in the longitudinal direction of the first relay board 55.

Each land portion 553 is connected with the connection connector 552 through the inside of the first relay board 55. A cable strand 511 at the other end of the cable unit 50 is, for example, soldered with each land portion 553. A connection between the cable strand 511 and the land portion 553 is potted with an insulating resin 557.

In a similar manner to the first relay board 55, the second relay board 253 also includes land portions 555 and a through hole 556. Each land portion 555 is connected with the connection connector 552 through the inside of the second relay board 253. Corresponding cable strands 252 are respectively soldered with the land portions 555. Since the first relay board 55 and the second relay board 253 each have the same shape, a detailed description of the second relay board 253 is omitted.

Further, the connection connector 552 is also connected with the land portion 555 through the inside of the second relay board 253. That is, each land portion 553 of the first relay board 55 is connected on a one-to-one basis with each land portion 555 of the second relay board 253 through the connection connector 552.

The first relay board 55 and the second relay board 253 are disposed to face each other such that surfaces, on which the land portions 553 and 555 are formed, face in opposite directions to each other. In this situation, the through hole 554 of the first relay board 55 and the through hole 556 of the second relay board 253 are aligned with each other in facing directions of the first relay board 55 and the second relay board 253. A screw 259 is inserted into the through hole 554 of the first relay board 55 and the through hole 556 of the second relay board 253, and is screwed into a screw hole formed in a frame 258 attached to the inside of the operation unit 20, and thus the first relay board 55 and the second relay board 253 are secured.

A unit hole 71, which penetrates in the axial length direction, is formed in the distal tip 13. The unit hole 71 is formed in a central portion of a half part of the distal tip surface 131 having a circular shape. The unit hole 71 has a quadrangular shape in a cross-sectional view, and the imaging device 15 is inserted into the unit hole 71. The observation window 132 is exposed from the distal tip surface 131.

In the housing tube 60, at an end portion of the circular hole portion 61, claw holes 619 are respectively formed on two outer surfaces facing each other. Further, a recess 682 is formed on one of such two outer surfaces, at an end portion of the quadrangular hole portion 68 (see Fig. 4).

The housing tube 60 is disposed such that the facing directions of the two outer surfaces, on which the claw holes 619 are formed, agree with a radial direction that passes an axial center of the observation window 132. In this situation, one surface, on which the recess 682 and the claw hole 619 are formed, is located closer to an edge than the other three surfaces.

A cable hole 42 is formed at a position corresponding to the unit hole 71, in the active bending section 12. The cable hole 42 has a circular shape in a cross-sectional view, and penetrates the active bending section 12 in the axial length direction. The cable hole 42 of the active bending section 12 communicates with the unit hole 71 of the distal tip 13, and the unit hole 71 and the cable hole 42 are coaxially arranged. The cable unit 50 is inserted into the cable hole 42.

A through hole (not illustrated) having the same diameter as the cable hole 42 of the active bending section 12 is formed in each of the passive bending section 11 and the bend preventing portion 16, and these through holes communicate with the cable hole 42. The cable unit 50 extends, through the through holes of the passive bending section 11 and the bend preventing portion 16, to the relay portion 40 of the operation unit 20.

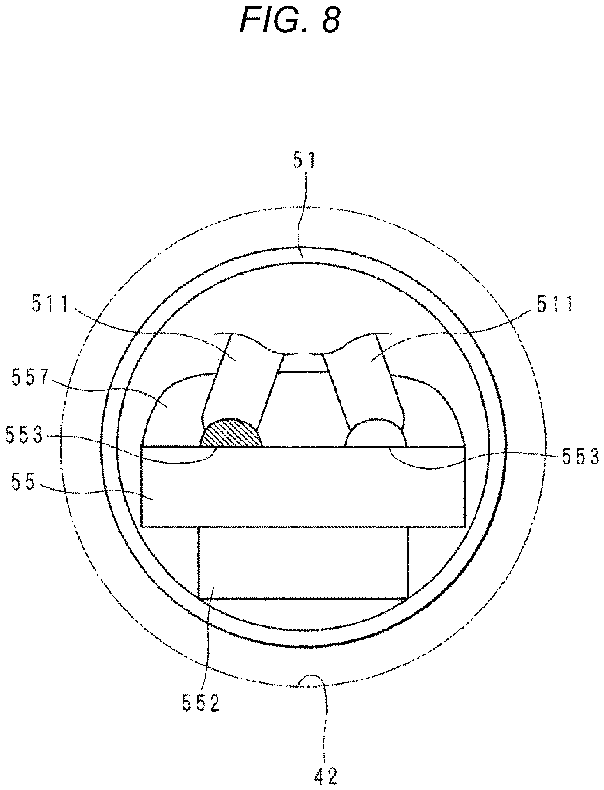

Fig. 8 is a diagram illustrating the size of the first relay board 55. For convenience, the cable hole 42 is indicated by a two-dot chain line, and a state in which the second relay board 253 is removed is displayed.

The diameter of the cable tube 51 is smaller than the diameter of the cable hole 42, and the dimension in the width direction of the first relay board 55 is smaller than the diameter of the cable tube 51. Therefore, the first relay board 55 is movable inside the cable hole 42 of the active bending section 12 and inside the through holes of the passive bending section 11 and the bend preventing portion 16.

On the distal tip surface 131 of the distal tip 13, illumination windows 133 are respectively provided interposing the observation window 132 in a direction orthogonal to the facing directions of the two outer surfaces of the housing tube 60. That is, in the distal tip 13, illumination holes 76, which penetrate the distal tip 13 in the axial length direction, are respectively formed on both sides of the unit hole 71. The illumination hole 76 has a circular shape in a cross-sectional view, and has one end opened on the distal tip surface 131, and the illumination window 133 is disposed at such one end.

A light emitting element 136 is inserted into each illumination hole 76, and the illumination window 133 expands an irradiation angle of the illumination light emitted from the light emitting element 136, and causes the illumination light to emit. Each light emitting element 136 is connected with an illumination cable 761 from the other end side of the illumination hole 76, and the illumination cable 761 supplies electric power to the light emitting element 136.

Illumination cable holes 46 are respectively formed at positions corresponding to the illumination holes 76, in the active bending section 12. The illumination cable hole 46 has a circular shape in a cross-sectional view, has a diameter smaller than that of the cable hole 42, and penetrates the active bending section 12 in the axial length direction. The illumination cable hole 46 of the active bending section 12 communicates with the illumination hole 76 of the distal tip 13, and the illumination hole 76 and the illumination cable hole 46 are coaxially arranged. The illumination cable 761 is inserted into the illumination cable hole 46.

Through holes (not illustrated) each having the same diameter as the illumination cable hole 46 of the active bending section 12 are formed in the passive bending section 11, and these through holes respectively communicate with the illumination cable holes 46. The illumination cable 761 extends, through the through holes of the passive bending section 11 and the bend preventing portion 16 and through the operation unit 20, to the connector unit 24.

In a substantially half part of the distal tip surface 131 of the distal tip 13, a substantially semicircular recess 75 is formed. Two illumination windows 133 and the observation window 132 are disposed at the bottom of the recess 75. That is, two illumination holes 76 and the unit hole 71 are opened at the bottom of the recess 75.

The recess 75 is covered with a window plate 145 having a semicircular shape that follows the recess 75. The front surface of the window plate 145 is flush with the distal tip surface 131. In addition, both end portions of a linearly extending edge in the window plate 145 are R-chamfered, and a gap is formed with a side wall of the recess 75.

In the remaining half part of the distal tip surface 131 and in the vicinity of one illumination window 133 of the two illumination windows 133, an air supply nozzle 143 and a water supply nozzle 144, which are used for cleaning or the like of the window plate 145 while an endoscopic examination is being conducted, and a jet outlet 141, which is used for washing away residues, mucus, blood, and the like from an observation target site, are provided.

Water or air that has been fed from an air supply/water supply device through a water supply hole (not illustrated) or an air supply hole (not illustrated) that passes through the universal cord 25, the active bending section 12, the passive bending section 11, and the bend preventing portion 16 is discharged from the water supply nozzle 144 or the air supply nozzle 143 toward the observation window 132 in accordance with an operation of the operation unit 20.

The diameter of the jet outlet 141 is reduced in the vicinity of the distal tip surface 131, and the water, which has been fed from the air supply/water supply device through the water supply hole (not illustrated) that penetrates the universal cord 25, the active bending section 12, the passive bending section 11, and the bend preventing portion 16, is vigorously discharged from the jet outlet 141.

A bending wire hole for a bending wire 17, which penetrates the insertion portion 14 in the axial length direction, and which is for an operation of bending the distal tip 13, is formed in the insertion portion 14. The bending wire hole includes a bending wire hole 47, which is formed in the active bending section 12, and bending wire holes, which are respectively formed in the passive bending section 11 and the bend preventing portion 16. The bending wire hole 47 of the active bending section 12 and the bending wire holes of the passive bending section 11 and the bend preventing portion 16 each have the same shape, and communicate with each other. For convenience, only the bending wire hole 47 will be described in the following.

The bending wire hole 47 has a circular shape in a cross-sectional view, and four bending wire holes 47 are formed in the vicinity of the outer circumferential surface of the active bending section 12. The bending wire holes 47 are formed to be separated at equal intervals in the circumferential direction. One end of the bending wire hole 47 is opened on an end surface of the active bending section 12, on the distal tip 13 side.

In the distal tip 13, a recess 77 is formed at a position corresponding to such one end of the bending wire hole 47, on an end surface on the active bending section 12 side. That is, on the end surface of the distal tip 13, the recesses 77 are formed at four locations at equal intervals in the circumferential direction. Each recess 77 has a circular shape in a cross-sectional view, and has a diameter larger than that of the bending wire hole 47. Each recess 77 is formed coaxially with the corresponding bending wire hole 47.

The bending wire 17 is inserted into each bending wire hole 47. One end of the bending wire 17 protrudes into the corresponding recess 77 through such one end of the bending wire hole 47. One end of the bending wire 17 is secured in the recess 77 by brazing, caulking, or the like. The other end of the bending wire 17 is connected with the bending knob 21 of the operation unit 20 through the bending wire hole. The user operates the bending knob 21 to cause the bending wire 17 to bend the active bending section 12.

In addition, as illustrated in Fig. 4, on the outer circumferential surface of the distal tip 13, a communication hole 712, which communicates with the unit hole 71, is formed at a position corresponding to the recess 682 of the housing tube 60 in the radial direction. The communication hole 712 has a circular shape in a cross-sectional view, and a screw thread is formed on the inner circumferential surface. A securing screw 69 is screwed into the communication hole 712. When the securing screw 69 comes into pressure contact with the housing tube 60, the position of the housing tube 60 in the unit hole 71 is secured.

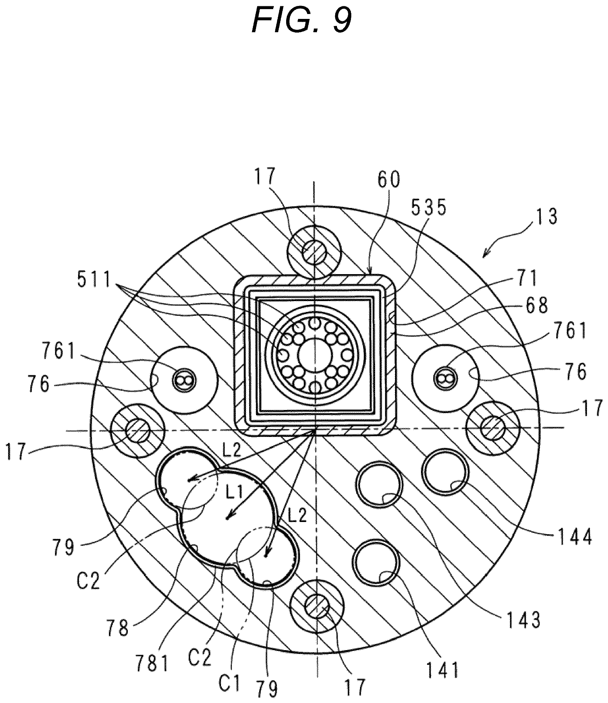

Fig. 9 is a cross-sectional view taken along line IX-IX in Fig. 4.

In the insertion portion 14, a treatment tool passage, which penetrates the insertion portion 14 in the axial length direction, and into which a treatment tool (see a two-dot chain line in Fig. 2) that has been inserted from the channel inlet 22 penetrates, is formed. The treatment tool passage includes a treatment tool passage 78, which is formed in the distal tip 13, a treatment tool passage 48, which is formed in the active bending section 12, and a treatment tool passage (not illustrated), which is formed in the passive bending section 11 (see Fig. 4). The treatment tool passage 78, the treatment tool passage 48, and the treatment tool passage of the passive bending section 11 each have a substantially circular shape in a cross-sectional view, and each have the same diameter. The treatment tool passage 78, the treatment tool passage 48, and the treatment tool passage of the passive bending section 11 each have a diameter larger than that of the treatment tool by 0.1 mm to 0.5 mm, for example.

One end (hereinafter, a channel outlet 142) of the treatment tool passage 78 is enlarged in diameter, and is opened in the vicinity of the other illumination window 133 of the two illumination windows 133, in the remaining half part of the distal tip surface 131. The other end of the treatment tool passage 78 communicates with one end of the treatment tool passage 48 through a channel pipe 781 (see Fig. 4).

That is, the channel pipe 781 is interposed between the distal tip 13 and the active bending section 12. One end of the channel pipe 781 is internally fit with the treatment tool passage 78, and the other end of the channel pipe 781 is internally fit with the treatment tool passage 48. The channel pipe 781 is disposed coaxially with the treatment tool passage 78 and the treatment tool passage 48. It is to be noted that the other end of the treatment tool passage 48 communicates with the treatment tool passage of the passive bending section 11.

In addition, two fluid passages 79, which penetrate the insertion portion 14 in the axial length direction, are formed in the insertion portion 14 (see Fig. 1). Each of the fluid passages 79 includes a through hole that has the same diameter and that penetrates the active bending section 12, the passive bending section 11, and the bend preventing portion 16, and these through holes communicate with each other. Furthermore, each of the fluid passages 79 has a substantially circular shape in a cross-sectional view, and has a diameter smaller than that of the treatment tool passage 78. One end (hereinafter, a fluid passage opening 791) of each fluid passage 79 is opened in the vicinity of the other illumination window 133 of the two illumination windows 133, in the remaining half part of the distal tip surface 131. A fluid such as a perfusate is sucked through the fluid passage opening 791, and flows to the operation unit 20 side through the fluid passage 79.

The two fluid passages 79 are formed to face each other in the radial direction of each of the treatment tool passage 78, the treatment tool passage 48, and the treatment tool passage of the passive bending section 11. The positional relationship with respect to the fluid passage 79 is the same in any of the treatment tool passage 78, the treatment tool passage 48, and the treatment tool passage of the passive bending section 11. In the following, only the treatment tool passage 78 will be described for convenience of description.

The treatment tool passage 78 and the two fluid passages 79 are formed to be partially continuous in a cross-sectional view. That is, as illustrated in Figs. 2 and 9, a circle C1 defined by the cross-section of the treatment tool passage 78 partially overlaps a circle C2 defined by the cross-section of each fluid passage 79, and the treatment tool passage 78 and the fluid passage 79 are integrated as a whole.

In other words, the treatment tool passage 78 and the two fluid passages 79 are integrated to form a single hole including two major arc-shaped parts relating to the fluid passages 79 and two minor arc-shaped parts relating to the treatment tool passage 78, in a cross-sectional view. The major arc-shaped parts and the minor arc-shaped parts face each other.

The distance from the axial center of the distal tip 13 (insertion portion 14) to each fluid passage 79, that is, a distance L2 from the axial center of the distal tip 13 to the center of the circle C2 related to the fluid passage 79 is equal to or longer than the distance from the axial center of the distal tip 13 (insertion portion 14) to the treatment tool passage 78, that is, equal to or longer than a distance L1 from the axial center of the distal tip 13 to the center of the circle C1 related to the treatment tool passage 78. In other words, the treatment tool passage 78 and the fluid passage 79 are formed at positions that are equal to each other from the outer circumferential surface of the distal tip 13, or the fluid passage 79 is formed at an outer position closer to the outer circumferential surface of the insertion portion 14 than the treatment tool passage 78.

From the endoscope 10 having the above configuration in the first embodiment, the imaging device 15, which is expensive, is collectable and reusable after it was used once.

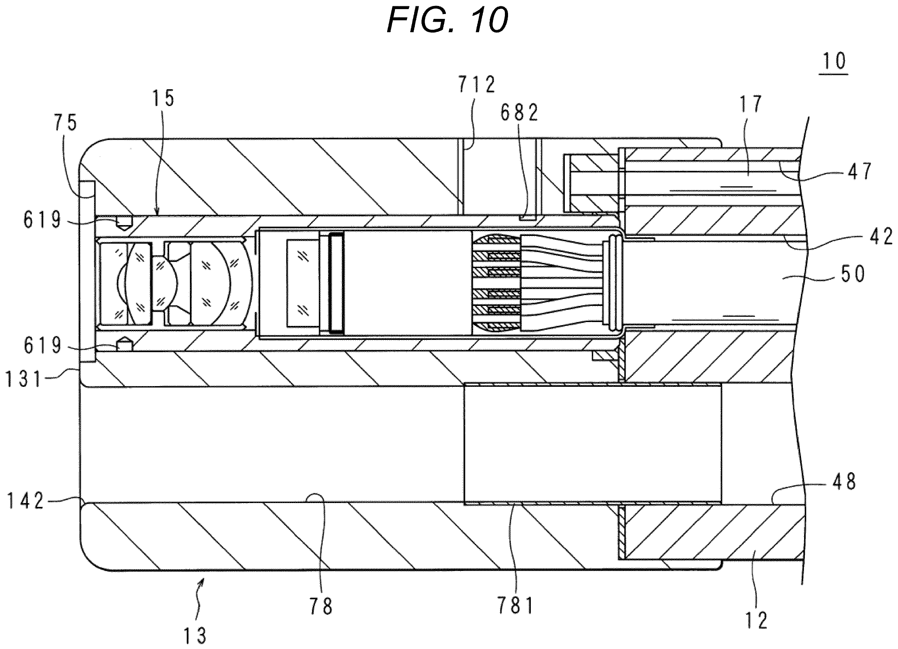

Figs. 10 to 13 are explanatory diagrams for describing a method for collecting the imaging device 15 from the endoscope 10. Hereinafter, collection of the imaging device 15 will be described in detail.

First, the operation unit 20 is disassembled, and the first relay board 55 is removed from the relay portion 40 (see Figs. 1 and 6). Next, a jig is inserted and gouged into a gap between the window plate 145 and the side wall of the recess 75, and thus the window plate 145 is removed from the distal tip 13 (see Fig. 2).

Next, the securing screw 69 is removed from the communication hole 712. Accordingly, as illustrated in Fig. 10, the recess 682 is exposed via the communication hole 712.

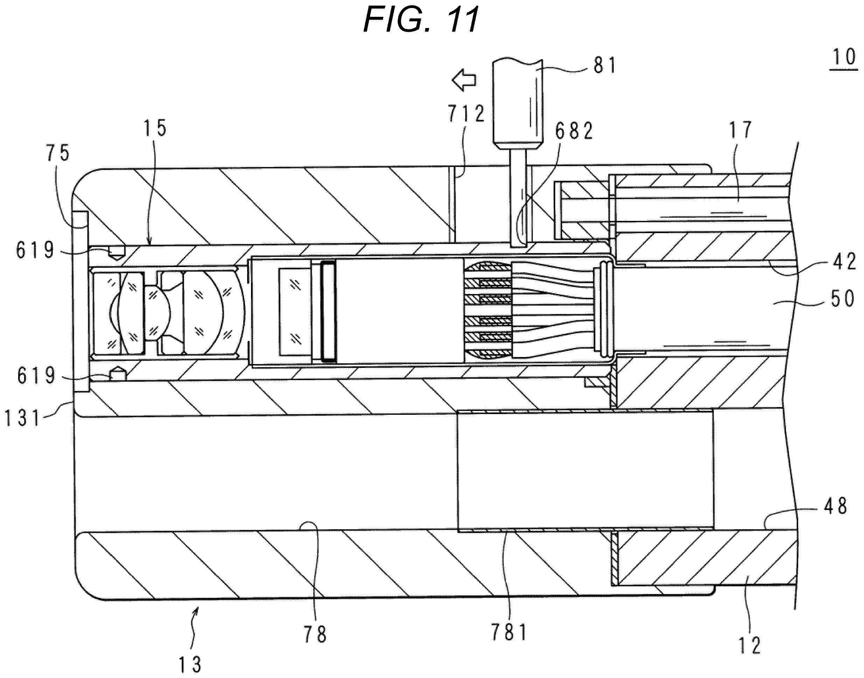

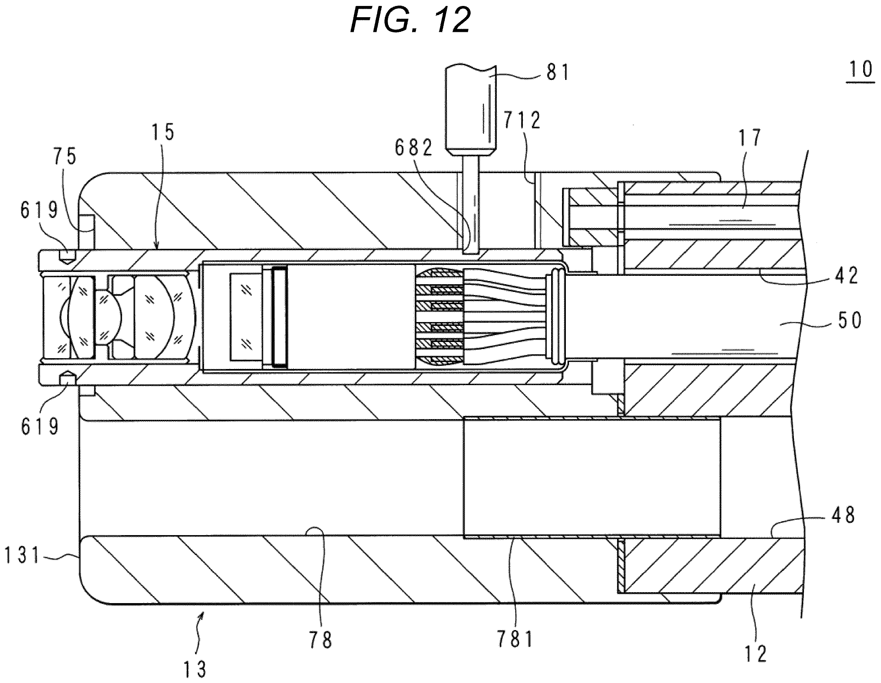

As illustrated in Fig. 11, an extrusion jig 81 having a rod-like tip is inserted into the recess 682 via the communication hole 712. The recess 682 is pushed out toward the distal tip side of the distal tip 13 (a direction of an arrow in Fig. 11) with use of the extrusion jig 81. Accordingly, as illustrated in Fig. 12, the distal tip of the imaging device 15 protrudes from the distal tip surface 131 of the distal tip 13, and the claw holes 619 are exposed.

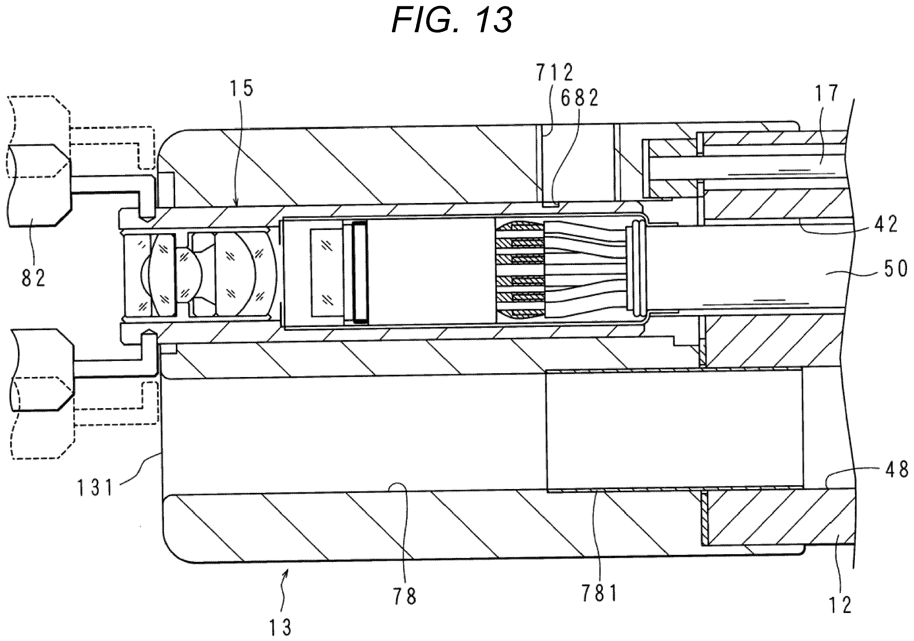

Then, as illustrated in Fig. 13, claw jigs 82 are respectively inserted into the two claw holes 619, the imaging device 15 is pulled out, and thus the imaging device 15 becomes removable from the endoscope 10. As described above, since the cable tube 51 and the first relay board 55 are smaller in diameter than the cable hole 42, the cable tube 51 and the first relay board 55 can be pulled out of the insertion portion 14 through the cable hole 42 and the unit hole 71.

With the above operation, the imaging device 15 is removable from the endoscope 10.

In addition, with the above configuration, the endoscope 10 in the first embodiment is capable of both improving the accuracy of the position control of the treatment tool and ensuring the gap for sucking the liquid.

As described above, the treatment tool passage 78 and the treatment tool passage 48 each have a diameter slightly larger than the diameter of the treatment tool. Therefore, the treatment tool is restrained in the treatment tool passage 78 and the treatment tool passage 48, and the movement of the treatment tool follows the movements of the treatment tool passage 78 and the treatment tool passage 48. Accordingly, the user operates the bending knob 21 to bend the active bending section 12, and is thus capable of controlling the direction of the treatment tool with high accuracy.

In addition, since the hole of the fluid passage 79 is always ensured independently of the treatment tool passage 78, the fluid such as the perfusate can be stably sucked.

Furthermore, as in choledochoscopic and cystoscopic examinations, also in a procedure performed while perfusing a liquid such as physiological saline, it is possible to perfuse the liquid sufficiently via the fluid passage 79, while the treatment tool being inserted into the treatment tool passage 78.

In addition, the treatment tool passage 78 and the fluid passage 79 are integrated to configure a single hole. Therefore, the configuration can be made compact as compared with a case where the treatment tool passage 78 and the fluid passage 79 are separately provided.

Further, in the endoscope 10 in the first embodiment, as described above, the fluid passage 79 is formed at an outer position closer to the outer circumferential surface of the insertion portion 14 than the treatment tool passage 78. Therefore, at the time of an endoscopic examination with use of the endoscope 10, it is possible to suck the liquid at an examination target site from a lower side. Therefore, the liquid easily flows along the gravity, and the liquid can be sucked more cleanly.

Heretofore, the description has been given for the case where the endoscope 10 includes the two fluid passages 79 as an example. However, the present invention is not limited to this, and a configuration including one fluid passage 79 may be adopted.

### (Second Embodiment)

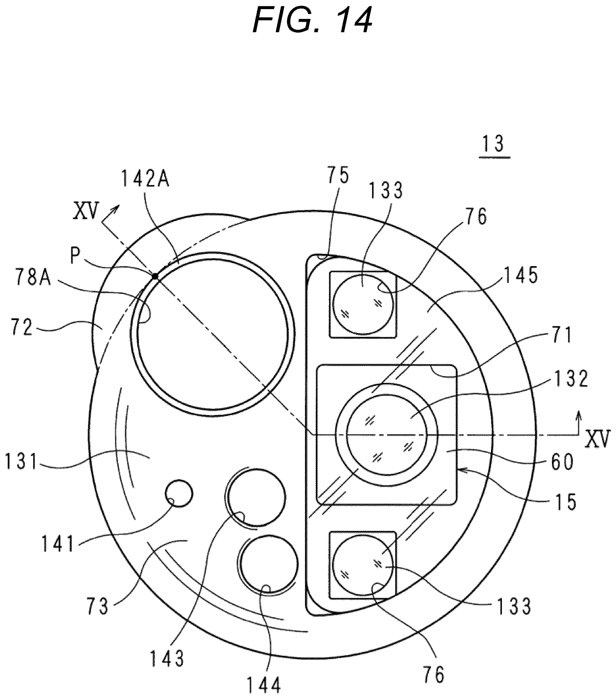

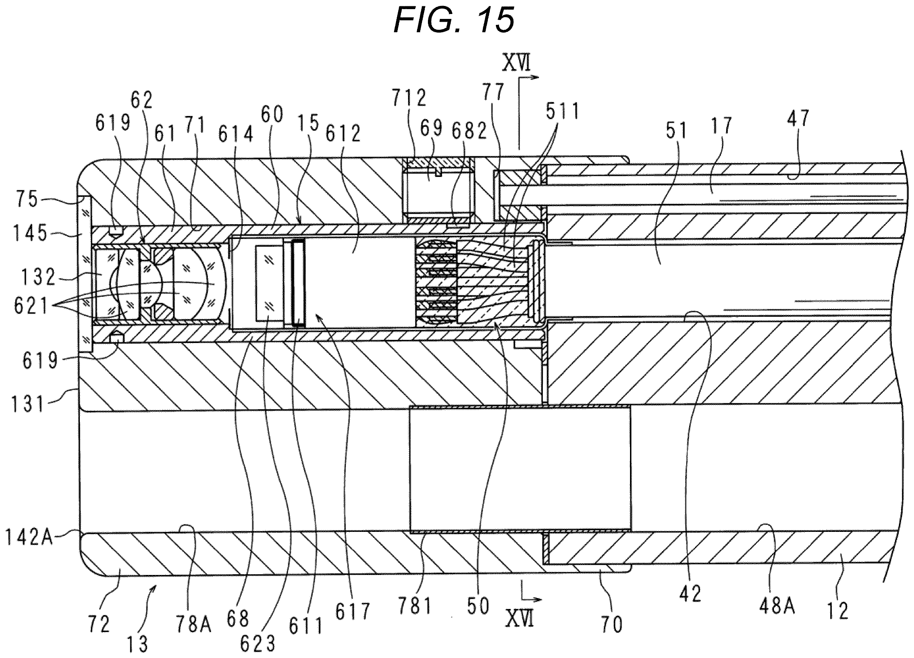

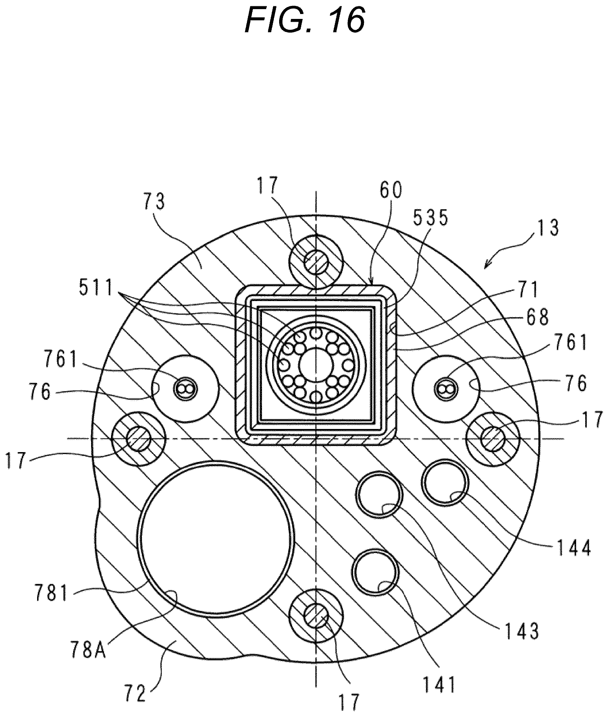

Fig. 14 is a diagram illustrating a distal tip surface 131 of a distal tip 13 in an endoscope 10 according to a second embodiment, Fig. 15 is a cross-sectional view taken along line XV-XV in Fig. 14, and Fig. 16 is a cross-sectional view taken along line XVI-XVI in Fig. 15.

In the endoscope 10 in the second embodiment, an insertion portion 14 includes a main portion 73 having a circular shape in a cross-sectional view, and an extension portion 72 extending in the radial direction at one position P on an outer circumference of the main portion 73. The extension portion 72 is formed over the entire length of the insertion portion 14. That is, in the insertion portion 14, the outer circumferential surface of the main portion 73 has a major arc shape in a cross-sectional view, and the outer circumferential surface of the extension portion 72 has a minor arc shape in a cross-sectional view.

In a substantially half part of the distal tip surface 131 of the distal tip 13, a substantially semicircular recess 75 is formed in a similar manner to the first embodiment. A unit hole 71, which penetrates the distal tip 13 in the axial length direction, is formed in the central part of the recess 75. The unit hole 71 has a quadrangular shape in a cross-sectional view, and the imaging device 15 is inserted into the unit hole 71. An observation window 132 of the imaging device 15 is exposed from the distal tip surface 131. In addition, at the bottom of the recess 75, two illumination windows 133 are disposed on both sides of the imaging device 15, and the observation window 132 is disposed.

Further, in the remaining half part of the distal tip surface 131 and in the vicinity of one illumination window 133 of the two illumination windows 133, an air supply nozzle 143 and a water supply nozzle 144, which are used for cleaning or the like of the window plate 145 while an endoscopic examination is being conducted, and a jet outlet 141, which is used for washing away residues, mucus, blood, and the like from an observation target site, are provided. Detailed descriptions of the same parts in the first embodiment will be omitted.

In the endoscope 10 in the second embodiment, a treatment tool passage (through passage), into which a treatment tool that has been inserted from the channel inlet 22 (see Fig. 1) penetrates, is formed at one position P. The treatment tool passage penetrates the insertion portion 14 in the axial length direction. The treatment tool passage includes a treatment tool passage 78A, which is formed in the distal tip 13, a treatment tool passage 48A, which is formed in the active bending section 12, and a treatment tool passage (not illustrated), which is formed in the passive bending section 11. The treatment tool passage 78A, the treatment tool passage 48A, and the treatment tool passage of the passive bending section 11 each have a substantially circular shape in a cross-sectional view, and each have the same diameter.

One end (hereinafter, a channel outlet 142A) of the treatment tool passage 78A is enlarged in diameter, and is opened in the vicinity of the other illumination window 133 of the two illumination windows 133 in the remaining half part of the distal tip surface 131. The other end of the treatment tool passage 78A communicates with one end of the treatment tool passage 48A through a channel pipe 781.

That is, the channel pipe 781 is interposed between the distal tip 13 and the active bending section 12, and the channel pipe 781 has a circular shape in a cross-sectional view. One end portion of the channel pipe 781 is internally fit with the treatment tool passage 78A, and the other end portion of the channel pipe 781 is internally fit with the treatment tool passage 48A. The channel pipe 781 is disposed coaxially with the treatment tool passage 78A and the treatment tool passage 48A. It is to be noted that the other end of the treatment tool passage 48A communicates with the treatment tool passage of the passive bending section 11.

Since the treatment tool passage 78A, the treatment tool passage 48A, and the treatment tool passage of the passive bending section 11 each have the same shape, only the treatment tool passage 78A will be described in the following.

As described above, the treatment tool passage 78A is formed at one position P. The treatment tool passage 78A is formed to include one position P, which is a position separated from the center of the main portion 73 by a radius of the main portion 73, and the center of the treatment tool passage 78A is located on a line connecting such one position P and the center of the main portion 73.

That is, in the extension portion 72, a position separated from the center of the main portion 73 by the radius of the main portion 73, in other words, a position on an extension line (one-dot chain line in Fig. 14) of the outer circumference of the main portion 73 is formed to be included in the treatment tool passage 78A (channel outlet 142A) (see Fig. 14). In Fig. 14, a case where the edge of the channel outlet 142A is located on the extension line of the outer circumference of the main portion 73 is viewed as an example. However, the present invention is not limited to this, and the treatment tool passage 78A may be provided such that the extension line of the outer circumference of the main portion 73 may be located on an inner side of the edge of the channel outlet 142A.

The treatment tool that has been inserted from the channel inlet 22 (see Fig. 1) comes out of the channel outlet 142A through the treatment tool passage of the passive bending section 11, the treatment tool passage 48A, and the treatment tool passage 78A, so that diagnosis, treatment, and the like are conducted in the body. In this situation, there is a gap between the treatment tool and the treatment tool passage 78A, the treatment tool passage 48A, and the treatment tool passage of the passive bending section 11. A liquid such as a body fluid in the body lumen can be sucked through such a gap.

In particular, in the endoscope 10 in the second embodiment, as described above, the treatment tool passage 78A, the treatment tool passage 48A, and the treatment tool passage of the passive bending section 11 are formed in a wide range including a position separated from the center of the main portion 73 by a radius of the main portion 73. Accordingly, a wide gap between the treatment tool and the treatment tool passage 78A, the treatment tool passage 48A, and the treatment tool passage of the passive bending section 11 can be ensured, and the liquid can be sucked stably.

Therefore, as in choledochoscopic and cystoscopic examinations, also in a procedure performed while perfusing a liquid such as physiological saline, it is possible to perfuse the liquid sufficiently.

In addition, in the endoscope 10 in the second embodiment, as described above, the treatment tool passage is formed in a wide range to the vicinity of the edge of the insertion portion 14. Thus, the extension portion 72 is provided for reinforcing the strength accordingly, and a thickness necessary for the strength between the treatment tool passage 78A, the treatment tool passage 48A, and the treatment tool passage of the passive bending section 11, and the outer circumferential surface of the insertion portion 14 is ensured.

That is, the diameter itself of the insertion portion 14 is not increased, but the extension portion 72 is provided only in a part where the treatment tool passage 78A, the treatment tool passage 48A, and the treatment tool passage of the passive bending section 11 are present, so that the insertion portion 14 is suppressed from becoming thick as much as possible. Accordingly, a burden on a subject who undergoes an endoscopic examination can be minimized.

Other configurations of the endoscope 10 in the second embodiment are the same as those of the endoscope 10 in the first embodiment, and the same components as those of the first embodiment are denoted by the same reference numerals, and detailed descriptions will be omitted.

### (Third Embodiment)

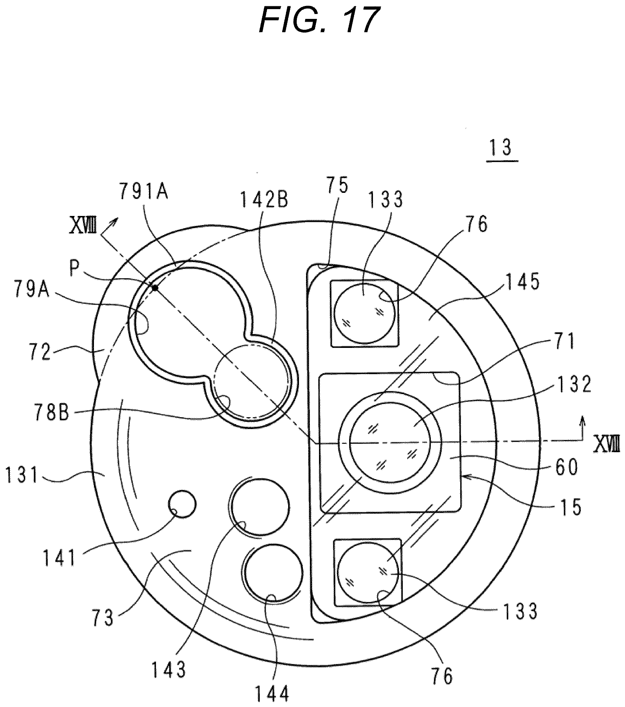

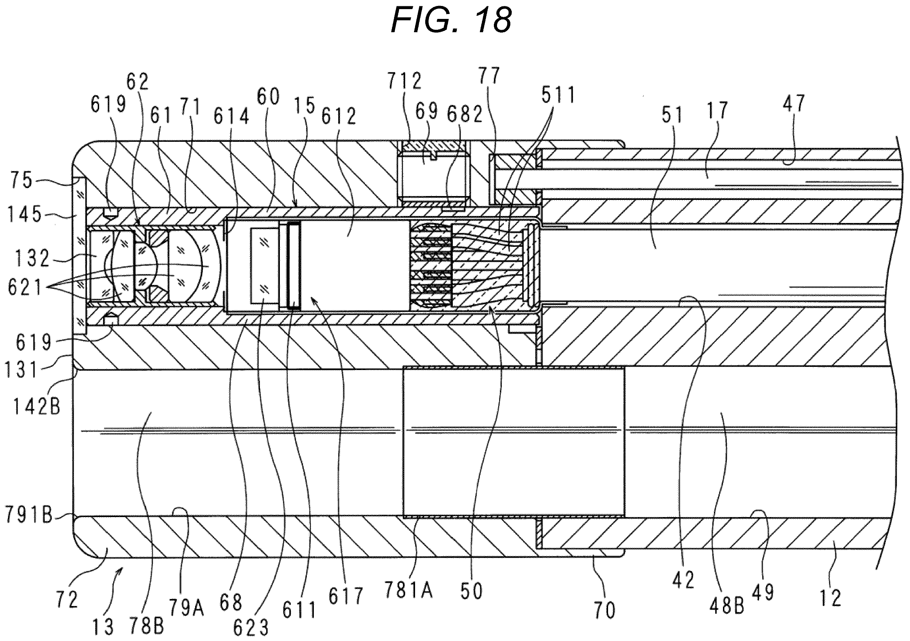

Fig. 17 is a diagram illustrating a distal tip surface 131 of a distal tip 13 in an endoscope 10 according to a third embodiment, and Fig. 18 is a cross-sectional view taken along line XVIII-XVIII in Fig. 17.

An insertion portion 14 includes a main portion 73 having a circular shape in a cross-sectional view, and an extension portion 72 extending in the radial direction at one position P on the outer circumference of the main portion 73. The extension portion 72 is formed over the entire length of the insertion portion 14. That is, in the insertion portion 14, the outer circumferential surface of the main portion 73 has a major arc shape in a cross-sectional view, and the outer circumferential surface of the extension portion 72 has a minor arc shape in a cross-sectional view.

In a substantially half part of the distal tip surface 131 of the distal tip 13, a recess 75 having a substantially semicircular shape is formed in a similar manner to the first embodiment, and a unit hole 71 is formed in a central portion of the recess 75. An imaging device 15 is inserted into the unit hole 71, and an observation window 132 of the imaging device 15 is exposed from the distal tip surface 131. At the bottom of the recess 75, two illumination windows 133 are disposed on both sides of the imaging device 15, and the observation window 132 is disposed.

Further, in the remaining half part of the distal tip surface 131 and in the vicinity of one illumination window 133 of the two illumination windows 133, an air supply nozzle 143, a water supply nozzle 144, and a jet outlet 141 are provided. Detailed descriptions of the same parts in the first embodiment will be omitted.

In the insertion portion 14, a treatment tool passage, which penetrates the insertion portion 14 in the axial length direction, and into which a treatment tool (see a two-dot chain line in Fig. 17) that has been inserted from the channel inlet 22 (see Fig. 1) penetrates, is formed. The treatment tool passage includes a treatment tool passage 78B, which is formed in the distal tip 13, a treatment tool passage 48B, which is formed in the active bending section 12, and a treatment tool passage (not illustrated), which is formed in the passive bending section 11. The treatment tool passage 78B, the treatment tool passage 48B, and the treatment tool passage of the passive bending section 11 each have a substantially circular shape in a cross-sectional view, and each have the same diameter. The treatment tool passage 78B, the treatment tool passage 48B, and the treatment tool passage of the passive bending section 11 each have a diameter larger than that of the treatment tool by 0.1 mm to 0.5 mm, for example.

One end (hereinafter, a channel outlet 142B) of the treatment tool passage 78B is enlarged in diameter, and is opened in the vicinity of the other illumination window 133 of the two illumination windows 133. The other end of the treatment tool passage 78B communicates with one end of the treatment tool passage 48B through a channel pipe 781A. It is to be noted that the other end of the treatment tool passage 48B communicates with the treatment tool passage of the passive bending section 11.

In addition, a fluid passage (through passage) that penetrates the insertion portion 14 in the axial length direction is formed in the insertion portion 14. The fluid passage includes a fluid passage 79A formed in the distal tip 13, a fluid passage 49 formed in the active bending section 12, and a fluid passage (not illustrated) formed in the passive bending section 11. The fluid passage 79A, the fluid passage 49, and the fluid passage of the passive bending section 11 each have a substantially circular shape in a cross-sectional view, and each have the same diameter. The fluid passage 79A, the fluid passage 49, and the fluid passage of the passive bending section 11 each have a diameter larger than that of the treatment tool passage.

One end (hereinafter, a fluid passage opening 791A) of the fluid passage 79A is enlarged in diameter, and is opened in the vicinity of the other illumination window 133 of the two illumination windows 133. The other end of the fluid passage 79A communicates with one end of the fluid passage 49 through the channel pipe 781A. It is to be noted that the other end of the fluid passage 49 communicates with the fluid passage of the passive bending section 11. A fluid such as a perfusate is sucked through the fluid passage opening 791A, and flows to the operation unit 20 side through the fluid passage 79A and the fluid passage 49.

As described above, the channel pipe 781A is interposed between the distal tip 13 and the active bending section 12, and the channel pipe 781A has a substantially letter "8" shape in a cross-sectional view. One end of the channel pipe 781A is internally fit with the treatment tool passage 78B and the fluid passage 79A, and the other end of the channel pipe 781A is internally fit with the treatment tool passage 48B and the fluid passage 49.

The treatment tool passage and the fluid passage are formed so as to be adjacent to each other. That is, the treatment tool passage 78B, the treatment tool passage 48B, and the treatment tool passage of the passive bending section 11 are respectively adjacent to the fluid passage 79A, the fluid passage 49, and the fluid passage of the passive bending section 11. The positional relationship between the treatment tool passage and the fluid passage is the same over the entire length of the insertion portion 14. In the following, only a positional relationship between the treatment tool passage 78B and the fluid passage 79A will be described for convenience of description.

The treatment tool passage 78B and the fluid passage 79A are formed to be partially continuous in a cross-sectional view. As illustrated in Fig. 17, a circle defined by the channel outlet 142B of the treatment tool passage 78B and a circle defined by the fluid passage opening 791A of the fluid passage 79A partially overlap each other. As a whole, the channel outlet 142B and the fluid passage opening 791A are integrated to form a substantially letter "8" shape.

In other words, the treatment tool passage 78B and the fluid passage 79A are integrated to form a single hole including a major arc-shaped part related to the fluid passage 79A and a major arc-shaped part related to the treatment tool passage 78B, in a cross-sectional view.

The distance from the axial center of the main portion 73 to the through passage is longer than the distance from the axial center of the main portion 73 to the treatment tool passage. That is, the distance from the axial center of the main portion 73 to the axial center of the fluid passage 79A is longer than the distance from the axial center of the main portion 73 to the axial center of the treatment tool passage 78B. In other words, the fluid passage 79A is formed at a position closer to the outer circumferential surface of the insertion portion 14 than the treatment tool passage 78B.

As described above, the fluid passage 79A is formed at one position P. The fluid passage 79A is formed to include one position P, which is a position separated from the center of the main portion 73 by a radius of the main portion 73, and the center of the fluid passage 79A is located on a line connecting such one position P and the center of the main portion 73.

That is, in the extension portion 72, a position separated from the center of the main portion 73 by the radius of the main portion 73, in other words, a position on an extension line (one-dot chain line in Fig. 17) of the outer circumference of the main portion 73 is formed to be included in the fluid passage 79A (fluid passage opening 791A) (see Fig. 17).

On the other hand, the center of the treatment tool passage 78B is located on a line connecting such one position P and the center of the main portion 73, and is located closer to the center of the main portion 73 than the center of the fluid passage 79A.

The treatment tool that has been inserted from the channel inlet 22 (see Fig. 1) comes out of the channel outlet 142B through the treatment tool passage of the passive bending section 11, the treatment tool passage 48B, and the treatment tool passage 78B, so that diagnosis, treatment, and the like are conducted in the body. In addition, a fluid such as a perfusate is sucked through the fluid passage opening 791A, and flows through the fluid passage 79A, the fluid passage 49, and the fluid passage of the passive bending section 11.

As described above, in the endoscope 10 in the third embodiment, the fluid passage 79A, the fluid passage 49, and the fluid passage of the passive bending section 11 are formed in a wide range including a position separated from the center of the main portion 73 by the radius of the main portion 73. Therefore, the fluid passage 79A, the fluid passage 49, and the fluid passage of the passive bending section 11 can be ensured widely, and the liquid can be sucked stably at any time.

Therefore, as in choledochoscopic and cystoscopic examinations, also in a procedure performed while perfusing a liquid such as physiological saline, it is possible to perfuse the liquid sufficiently into the fluid passage, while inserting the treatment tool into the treatment tool passage.

In addition, in the endoscope 10 in the third embodiment, the fluid passage is formed in a wide range to the vicinity of the edge of the insertion portion 14 as described above. Thus, the extension portion 72 is provided for reinforcing the strength accordingly, and a thickness necessary for the strength between the fluid passage 79A, the fluid passage 49, and the fluid passage of the passive bending section 11, and the outer circumferential surface of the insertion portion 14 is ensured.

That is, the diameter itself of the insertion portion 14 is not increased, but the extension portion 72 is provided only in a part where the fluid passage 79A, the fluid passage 49, and the fluid passage of the passive bending section 11 are present, so that the insertion portion 14 is suppressed from becoming thick as much as possible. Accordingly, a burden on a subject who undergoes an endoscopic examination can be minimized.

In addition, in the endoscope 10 in the third embodiment, as described above, the treatment tool passage 78B, the treatment tool passage 48B, and the treatment tool passage of the passive bending section 11 (hereinafter, simply a treatment tool passage) each have a diameter slightly larger than the diameter of the treatment tool. Therefore, the treatment tool is restrained in the treatment tool passage, and the movement of the treatment tool follows the movement of the treatment tool passage. Accordingly, the user operates the bending knob 21 (see Fig. 1) to bend the active bending section 12, and is thus capable of controlling the direction of the treatment tool with high accuracy.

In addition, in the endoscope 10 in the third embodiment, as described above, the fluid passage is formed at a position closer to the outer circumferential surface of the insertion portion 14 than the treatment tool passage. Therefore, at the time of an endoscopic examination with use of the endoscope 10, it is possible to suck the liquid at an examination target site from a lower side. Therefore, the liquid easily flows along the gravity, and the liquid can be sucked more cleanly.

Other configurations of the endoscope 10 in the third embodiment are the same as those of the endoscope 10 in the first embodiment, and the same components as those of the first embodiment are denoted by the same reference numerals, and detailed descriptions will be omitted.

### (Fourth Embodiment)

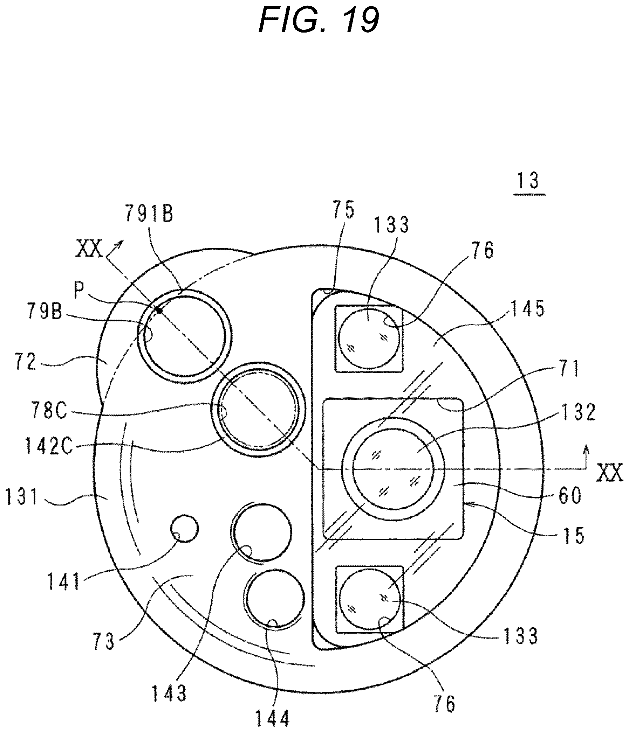

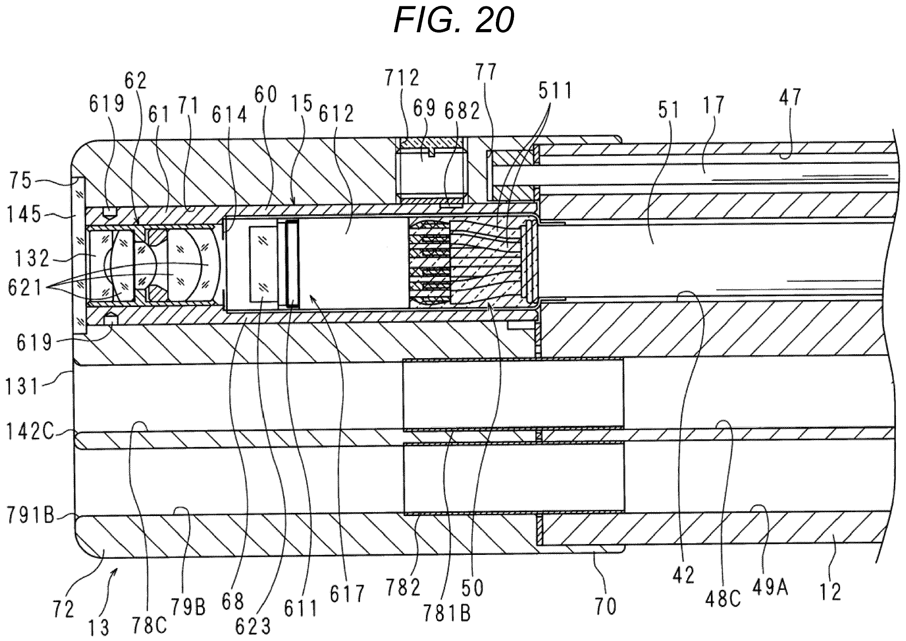

Fig. 19 is a diagram illustrating a distal tip surface 131 of a distal tip 13 in an endoscope 10 according to a fourth embodiment, and Fig. 20 is a cross-sectional view taken along line XX-XX in Fig. 19.

In the endoscope 10 in the fourth embodiment, in a similar manner to the second and third embodiments, an extension portion 72 extending outward in the radial direction from the main portion 73 is formed in the insertion portion 14 over the entire length.

In a substantially half part of the distal tip surface 131 of the distal tip 13, a recess 75 having a substantially semicircular shape is formed in a similar manner to the first embodiment, and a unit hole 71 is formed in a central portion of the recess 75. An imaging device 15 is inserted into the unit hole 71, and an observation window 132 of the imaging device 15 is exposed from the distal tip surface 131. At the bottom of the recess 75, two illumination windows 133 are disposed on both sides of the imaging device 15, and the observation window 132 is disposed.

Further, in the remaining half part of the distal tip surface 131 and in the vicinity of one illumination window 133 of the two illumination windows 133, an air supply nozzle 143, a water supply nozzle 144, and a jet outlet 141 are provided. Detailed descriptions of the same parts in the first embodiment will be omitted.

In the insertion portion 14, a treatment tool passage, which penetrates the insertion portion 14 in the axial length direction, and into which a treatment tool (see a two-dot chain line in Fig. 19) that has been inserted from the channel inlet 22 (see Fig. 1) penetrates, is formed. The treatment tool passage includes a treatment tool passage 78C, which is formed in the distal tip 13, a treatment tool passage 48C, which is formed in the active bending section 12, and a treatment tool passage (not illustrated), which is formed in the passive bending section 11. The treatment tool passage 78C, the treatment tool passage 48C, and the treatment tool passage of the passive bending section 11 each have a substantially circular shape in a cross-sectional view, and each have the same diameter. The treatment tool passage 78C, the treatment tool passage 48C, and the treatment tool passage of the passive bending section 11 each have a diameter larger than that of the treatment tool by 0.1 mm to 0.5 mm, for example.

One end (hereinafter, a channel outlet 142C) of the treatment tool passage 78C is enlarged in diameter, and is opened in the vicinity of the other illumination window 133 of the two illumination windows 133. The other end of the treatment tool passage 78C communicates with one end of the treatment tool passage 48C through a channel pipe 781B. It is to be noted that the other end of the treatment tool passage 48C communicates with the treatment tool passage of the passive bending section 11.

In addition, a fluid passage (through passage) that penetrates the insertion portion 14 in the axial length direction is formed in the insertion portion 14. The fluid passage includes a fluid passage 79B formed in the distal tip 13, a fluid passage 49A formed in the active bending section 12, and a fluid passage (not illustrated) formed in the passive bending section 11. The fluid passage 79B, the fluid passage 49A, and the fluid passage of the passive bending section 11 each have a circular shape in a cross-sectional view, and each have the same diameter. The fluid passage 79B, the fluid passage 49A, and the fluid passage of the passive bending section 11 each have a diameter larger than that of the treatment tool passage.

One end (hereinafter, a fluid passage opening 791B) of the fluid passage 79B is enlarged in diameter, and is opened in the vicinity of the other illumination window 133 of the two illumination windows 133. The other end of the fluid passage 79B communicates with one end of the fluid passage 49A through a fluid passage pipe 782. It is to be noted that the other end of the fluid passage 49A communicates with the fluid passage of the passive bending section 11. A fluid such as a perfusate is sucked through the fluid passage opening 791B, and flows to the operation unit 20 side through the fluid passage 79B and the fluid passage 49A.

As described above, the channel pipe 781B and the fluid passage pipe 782 are interposed between the distal tip 13 and the active bending section 12, and the channel pipe 781B and the fluid passage pipe 782 each have a circular shape in a cross-sectional view. One end of the channel pipe 781B is internally fit with the treatment tool passage 78C, and the other end of the channel pipe 781B is internally fit with the treatment tool passage 48C. In addition, one end of the fluid passage pipe 782 is internally fit with the fluid passage 79B, and the other end of the fluid passage pipe 782 is internally fit with the fluid passage 49A.

The treatment tool passage 78C, the treatment tool passage 48C, and the treatment tool passage of the passive bending section 11 each have the same shape, and the fluid passage 79B, the fluid passage 49A, and the fluid passage of the passive bending section 11 each have the same shape. In the following, for convenience of description, only the treatment tool passage 78C and the fluid passage 79B will be described.

The distance from the axial center of the main portion 73 to the through passage is longer than the distance from the axial center of the main portion 73 to the treatment tool passage. That is, the distance from the axial center of the main portion 73 to the axial center of the fluid passage 79B is longer than the distance from the axial center of the main portion 73 to the axial center of the treatment tool passage 78C. In other words, the fluid passage 79B is formed at a position closer to the outer circumferential surface of the insertion portion 14 than the treatment tool passage 78C.

As illustrated in Fig. 19, the fluid passage 79B is formed at one position P. The fluid passage 79B is formed to include one position P, which is a position separated from the center of the main portion 73 by a radius of the main portion 73, and the center of the fluid passage 79B is located on a line connecting such one position P and the center of the main portion 73.

That is, in the extension portion 72, a position separated from the center of the main portion 73 by the radius of the main portion 73, in other words, a position on an extension line (one-dot chain line in Fig. 19) of the outer circumference of the main portion 73 is formed to be included in the fluid passage 79B (fluid passage opening 791B) (see Fig. 19).

On the other hand, the center of the treatment tool passage 78C is located on a line connecting such one position P and the center of the main portion 73, and is located closer to the center of the main portion 73 than the center of the fluid passage 79B.

The treatment tool that has been inserted from the channel inlet 22 (see Fig. 1) comes out of the channel outlet 142C through the treatment tool passage of the passive bending section 11, the treatment tool passage 48C, and the treatment tool passage 78C, so that diagnosis, treatment, and the like are conducted in the body. In addition, a fluid such as a perfusate is sucked through the fluid passage opening 791B, and flows through the fluid passage 79B, the fluid passage 49A, and the fluid passage of the passive bending section 11.

As described above, in the endoscope 10 in the fourth embodiment, the fluid passage 79B, the fluid passage 49A, and the fluid passage of the passive bending section 11 are formed in a wide range including a position separated from the center of the main portion 73 by the radius of the main portion 73. Therefore, the fluid passage 79B, the fluid passage 49A, and the fluid passage of the passive bending section 11 can be ensured widely, and the liquid can be sucked stably at any time.

Therefore, as in choledochoscopic and cystoscopic examinations, also in a procedure performed while perfusing a liquid such as physiological saline, it is possible to perfuse the liquid sufficiently into the fluid passage, while inserting the treatment tool into the treatment tool passage.

In addition, also in a procedure of performing high-frequency cauterization or laser irradiation, it is possible to suck smoke generated at the time of cauterization through the fluid passage, while inserting the treatment tool into the treatment tool passage. Therefore, it is possible to give treatment safely, while a visual field is being ensured.

In addition, in the endoscope 10 in the fourth embodiment, as described above, the fluid passage is formed in a wide range to the vicinity of the edge of the insertion portion 14. Thus, the extension portion 72 is provided for reinforcing the strength accordingly, and a thickness necessary for the strength between the fluid passage 79B, the fluid passage 49A, and the fluid passage of the passive bending section 11, and the outer circumferential surface of the insertion portion 14 is ensured.

That is, the diameter itself of the insertion portion 14 is not increased, but the extension portion 72 is provided only in a part where the fluid passage 79B, the fluid passage 49A, and the fluid passage of the passive bending section 11 are present, so that the insertion portion 14 is suppressed from becoming thick as much as possible. Accordingly, a burden on a subject who undergoes an endoscopic examination can be minimized.

In addition, in the endoscope 10 in the fourth embodiment, as described above, the fluid passage is formed at a position closer to the outer circumferential surface of the insertion portion 14 than the treatment tool passage. Therefore, at the time of an endoscopic examination with use of the endoscope 10, it is possible to suck the liquid at an examination target site from a lower side. Therefore, the liquid easily flows along the gravity, and the liquid can be sucked more cleanly.

Other configurations of the endoscope 10 in the fourth embodiment are the same as those of the endoscope 10 in the first embodiment, and the same components as those of the first embodiment are denoted by the same reference numerals, and detailed descriptions will be omitted.

Technical features (constitutional requirements) that have been described in the first to fourth embodiments can be combined with one another, and a new technical feature can be formed with the combination.

The embodiments that have been disclosed herein should be considered to be exemplary in all respects without being limited. The scope of the present invention is indicated by the scope of claims, not the above-mentioned meaning, and is intended to include all modifications within the meaning and scope equivalent to the claims.

### Reference Signs List

10 Endoscope
14 Insertion portion
13 Distal tip
48, 78 Treatment tool passage
79 Fluid passage
131 Distal tip surface
142 Channel outlet
L1, L2 Distance
791 Fluid passage opening
48A, 48B, 48C, 78A, 78B, 78C Treatment tool passage
49,49A, 79A, 79B Fluid passage
72 Extension portion
73 Main portion
142A, 142B, 142CChannel outlet
791A, 791B Fluid passage opening.

## Claims

1. An endoscope including an insertion portion having a round tubular shape to be inserted into a body lumen, the endoscope comprising:
a treatment tool passage having one end opened on a distal tip surface of the insertion portion, penetrating the insertion portion in an axial length direction,
and allowing a treatment tool to pass through; and
a fluid passage having one end opened on the distal tip surface of the insertion portion, penetrating the insertion portion in the axial length direction, and
allowing a fluid that has flowed from the one end to flow through, wherein
the treatment tool passage and the fluid passage are partially continuous in a cross-sectional view.

2. The endoscope according to claim 1, wherein
the treatment tool passage and the fluid passage each have a circular shape in the cross-sectional view, and
the treatment tool passage is larger in diameter than the fluid passage.

3. The endoscope according to claim 1, wherein a distance from an axial center of the insertion portion to the fluid passage is equal to or longer than a distance from the axial center of the insertion portion to the treatment tool passage.

4. An endoscope including an insertion portion having a tubular shape to be inserted into a body lumen, the endoscope comprising:
a main portion having a circular shape in a cross-sectional view;
an extension portion extending in a radial direction at one position of an outer circumference of the main portion; and
a through passage at the one position, the through passage penetrating the insertion portion in an axial length direction.

5. The endoscope according to claim 4, wherein the through passage includes a position that is the one position and that is separated from a center of the main portion by a radius.

6. The endoscope according to claim 4 or 5, wherein
one end of the through passage is opened on a distal tip surface of the insertion portion, and
a fluid is sucked through the one end of the through passage.

7. The endoscope according to claim 6, further comprising
a treatment tool passage having one end opened on the distal tip surface of the insertion portion, penetrating the insertion portion in an axial length direction, and allowing a treatment tool to pass through, wherein
a distance from an axial center of the main portion to the through passage is longer than a distance from the axial center of the main portion to the treatment tool passage.
